# EUROPEAN PATENT APPLICATION

(11) **EP 2 803 723 A1**
(43) Date of publication of application: **19.11.2014**
(21) Application number: 13168361.7
(22) Date of filing: 17.05.2013
(51) Int. Cl.: C12N 5/00

(54) **Cell cultivation scaffold**

(71) Applicant: Centre de Recherche Public Henri Tudor, 1855 Luxembourg (LU)
(72) Inventor: Pilarczyk, Götz, 68542 Heddesheim (DE); Lemor, Robert, 66121 Saarbrücken (DE)
(74) Representative: Watterson, Peer Marten John

(57) **Abstract**

A modular element, adapted to serve as a scaffold for cell cultivation, comprises a degradable, preferably biodegradable, thermoplastic sheet and a support structure therefor, said degradable sheet having a microstructure on one or both sides, and wherein said modular element is stackable, thereby permitting assembling of the modules into a structure resembling a tissue or organ sample, with eventual removal of the sheet and support. Such modules can easily be manufactured and handled.

## Description

### Technical Field

The present invention relates to a modular element for use in cell cultivation and, in particular, structures comprising a plurality of such modular elements, and a method of constructing such modular elements and structures.

### Background

Structures used for cell cultivation are known. It is also known to provide patterned structures whereby the pattern influences cell cultivation such that a desired cell arrangement may be obtained after cultivation.

Many different methods for providing such patterned structures are known. Such methods include soft lithography, self assembly, vapour deposition and photolithography. All of these methods form microscopic features on a substrate. Cell cultivation and differentiation is then encouraged on the substrate by the presence of these microstructures. Such microstructures may include microfluidic channels.

WO 2010/042856 and WO 2011/102991 briefly mention the possibility of constructing larger structures with the use of such patterned substrates, discussing the possibility of forming tissues, for example. There is no disclosure as to how such tissues or structures might be implemented.

Moreover, even if the tissues and structures were capable of being constructed in the manner described, such arrangements suffer from the disadvantage that, due to the manner in which they are formed, the tissues or structures are formed without any significant supporting structures. Therefore, the ability to investigate the operation and interaction of such tissues or structures with an environment resembling the environment in which they naturally occur is limited.

Approaches which use permanent or transient scaffolds as supporting structures are also known, but such structures comprise random geometries. Consequently, the effect of such scaffolds is similar to scaffold-free approaches: the support does not encourage geometric organisation.

Microlithography comprises covering a flat silicon surface with a caustic-resistant coating. This is followed by the partial removal of the coating following exposure by laser writing or by projective/contact exposure, leaving a surface pattern that is converted into a raised pattern by means of silicon etching. The surface patterns that can be obtained in this matter are immediately available for colonisation by adhering cultured cells. However, the process is time-consuming, requires a demanding infrastructure, such as clean procedures, and produces large amounts of hazardous waste, such as cerium and fluoride compounds, even for small operations. In addition, the method is not redundant; one microlithographic operation produces only one usable surface. Further, the structured surfaces are opaque, and are thus not suitable for light microscopy, which makes result checking difficult.

Etched glass is not an acceptable solution, as the etched glass surfaces are rough and interfere with the optical image. A combination of microlithography and a multi-step moulding process can overcome the limitations described above. In a first step, the moulding is carried out in a flexible polymer that polymerises on the silicon structure. Polydimethylsiloxane (PDMS) is a frequently used copolymer. The resulting stamp can be moulded again, e.g., in PDMS, following appropriate modification of its surface. If a flat glass substrate is selected as the base for the second moulding, substrates with a surface structure identical to the silicon substrate will be obtained that simultaneously are biocompatible and optically observable. The processes necessary for the production of superhydrophobic low-energy separation planes by means of condensative coupling of halogenated hydrocarbon silanes are known to those skilled in the art. This method also has disadvantages. The low aqueous wettability of PDMS is an obstacle to the adhesion of mature cells, thus reducing biocompatibility. Its chemical inertness makes modifications to improve this difficult. The combination of several chemically different soft polymers in a mosaic is only possible at a spatial resolution inadequate for biomedical requirements. Furthermore, the possible soft polymers are not sufficiently differentiated in order to be useable. PDMS is not biodegradable. Thus, the support structure remains permanently within the cell population, which results in an artificial tissue with a polymer content after the formation of tissue blocks. Free-floating cell plates are not available, as the cell plates, PDMS, support structures, and glass supports cannot be separated without damaging the cell plate. Thus, these preparations are not available for methods such as cell sheet engineering.

An alternative to moulding in PDMS is the production of a lamella using fused deposition modelling (FDM), a method known as rapid prototyping (RT). In FDM, a melted polymer is pressed through a fine nozzle, and hardens either as a point or as a string deposited in free form. A controller that coordinates the outlet nozzle and the movement and casting of the polymer allows for the construction of 3D bodies of polymers. This method also has limitations. In casting the polymer, the spatial resolution is limited by the diameter of the nozzle used. The nozzle diameter cannot be reduced to the levels recommended for cellular biology, which are in the single-figure micrometre range. Even if it were technically feasible, casting times would be unacceptably high.

WO2008/045506 discloses a method for creating biopolymer structures comprising providing a transitional polymer on a substrate; depositing a biopolymer on the transitional polymer; shaping the biopolymer into a structure having a selected pattern on the transitional polymer; and releasing the biopolymer from the transitional polymer. The resulting scaffolds are flimsy and difficult to handle, and releasing them from the transitional polymer is tricky and not given to commercialisation.

We have now found that it is possible to create patterned scaffolds for organised cell growth by microlithography of silicon, for example, creating a die by casting a hardenable moulding substance, such as PDMS on the microlithographically obtained template, and then obtaining the patterned scaffolds by heat stamping a thermoplastic sheet with the heated die.

### Summary of the Invention

According to a first aspect, the invention provides a modular element for cell cultivation, the modular element comprising a sheet having a microstructure formed thereon, wherein one or more modular elements further comprises one or more macrostructures, the macrostructure comprising a macrofeature wherein a dominant dimension of the macrofeature is of a similar order of magnitude as the sheet.

In an alternative aspect, there is provided a modular element, adapted to serve as a scaffold for cell cultivation, comprising a degradable, preferably biodegradable, thermoplastic sheet and a support structure therefor, said degradable sheet having a microstructure on one or both sides, and wherein said modular element is stackable.

A modular element, adapted to serve as a scaffold for cell cultivation, comprises a degradable, preferably biodegradable, thermoplastic sheet and a support structure therefor, said degradable sheet having a microstructure on one or both sides, and wherein said modular element is stackable, thereby permitting assembling of the modules into a structure resembling a tissue or organ sample, with eventual removal of the sheet and support. Such modules can easily be manufactured and handled.

A modular element of the present invention is also referred to herein as a module, and is stackable with other modules of the invention. By 'stackable' is meant that modules of the invention may be associated in such a manner that a plurality may be secured or arranged such that all or a portion of the degradable sheets may be brought into proximity, preferably such as to allow cell cultures associated with the sheets to form a contiguous cellular mass in the event of continued cellular growth.

The support structure is preferably in the form of a surround, or frame, for the sheet, with the sheet corresponding to a cross-section of a tissue or organ. By selecting appropriate cross-sections and cell cultures, modules of the invention can be arranged, or stacked, such that the sheets and their corresponding cell cultures form the shape of the tissue mass desired. Continued growth and any induced differentiation of the cells, and subsequent degradation of the sheets and removal of the supports eventually yields a structured tissue or part or all of an organ.

The term 'thermoplastic' has the meaning given to it in the art. The advantage of using a thermoplastic sheet is that it is possible to create the microstructure thereon by microlithography, for example. A heated die treated, if necessary, with a release coating to ensure that the die does not stick to the sheet, is pressed onto the sheet to create the microstructure, and then removed. It is preferred that this is done with the sheet already supported by the support, so that the resulting module is ready to use after pressing. Examples of treating the die and pressing methods are described further, hereinbelow. The advantage of such a structure is that it is both easy to manufacture in bulk, and is easy to handle.

The term 'scaffold' is used herein to designate a substance on which cells in culture can grow, or a support, and which preferably encourages any resulting cell mass to adopt a predetermined structure. For example, a scaffold comprising parallel channels, such as those described hereinbelow, may encourage cells to grow in parallel lines, such as might be found in muscle tissue.

The term 'degradable' is used herein to indicate that a substance can be degraded in the presence of cells from an associated cell culture such as to be able to separate the substance from any cell mass, or that the substance can be decomposed and thereby removed. This may be achieved naturally, or by the addition of degradative substances, such as enzymes or chemicals known to degrade the material in question, preferably without having any detrimental effect on the associated cells.

The term 'biodegradable' is used herein to indicate that a substance decomposes, or degrades, in the presence of water, or selected cell cultures, or all cell cultures, as desired, preferably any cell culture. Some substances, such as polyanilides, may decompose in the presence of water, and are only suitable where a scaffold is only needed for a short period of time, and possibly where no growth scaffold is required. In general, substances intended to be used as scaffolds preferably provide support for cell growth for at least a day, and up to a month, with between one week and two weeks being preferred.

Polylactic acid (PLA) decomposes in the body over a period about 18 months, with ∼50% being degraded over about 5 months, and is a preferred biodegradable polymer for use in the present invention. Where it is desired to reduce the period of degradation, the rate may be accelerated by copolymerising the PLA with a comonomer such as glycolic acid (GA) in ratios of up to about 90 : 1 GA: PLA. In the alternative, sterically hindering comonomers, may be used to reduce the rate of degradation still further.

The sheet may be constructed from at least a first polymer. The sheet is preferably formed from a thermoplastic.

Biodegradable plastics include: aliphatic polyesters such as polyhydroxyalkanoates (PHAs), for example poly-3-hydroxybutyrate (PHB), polyhydroxyvalerate (PHV) and polyhydroxyhexanoate (PHH); polylactic acid (PLA); polybutylene succinate (PBS); polycaprolactone (PCL); polyanhydrides; polyvinyl alcohol (PVA); starch derivatives; and thermoplastic cellulose esters, such as cellulose acetate, cellulose acetate butyrate, cellulose propionate, and ethyl cellulose. Preferred are PLA, poly(lactic-co-glycolic acid) (PLGA), Polylactic Acid and Polycaprolactone (PLA-PCA), PLA-polyurethane (PLA-PU), and any copolymers thereof.

In one embodiment, the material of the sheet is transparent, in a manner sufficient to allow observation by microscope of the cells in culture through the sheet.

The sheet is preferably biodegradable. Preferably, the sheet is biodegradable in a manner that is substantially or completely non-toxic to the cell culture or subsequent cell culture or tissue formation resulting from the culture. By non-toxic is meant that cells are not killed by the material of the sheet or its metabolites, and preferably does not slow growth to an extent of more than 90%, and preferably not more than 50% at any given time. Preferably there is no interference with growth. It will also be appreciated that the biodegradable material may comprise nutrients and other factors that may be released during decomposition.

Decomposition of the material of the sheet may be by the action of the cells in the cell medium, or by simple dissolution or other action, typically associated with the cell medium or culture.

In one embodiment, it is preferred that the material of the sheet eventually disappears leaving substantially no physical structural trace of its existence. In an alternative embodiment, a scaffold material, such as collagen, is embedded in the material, and remains after decomposition of the remainder of the material in order to assist in conferring a 3D scaffold for the cell mass formed by cultivation of the cell culture.

In another embodiment, the sheet is degraded only sufficiently that the cell mass can be floated free of the scaffold. The resulting cell mass, which may be a monolayer, for example, may then be used as desired.

The sheet carries a microstructure on one or both sides. This microstructure is preferably adapted for use in directing, at least to some extent, growth of any cells in cell culture to which the microstructure is exposed. It is preferred that the dimensions of the microstructure are such as to encourage attachment or embedding of cells in culture, and a preferred microstructure presents pits, elongated pits, or channels in which cells may be accommodated.

The pits or channels, also referred to herein as grooves, may be in raised structures on the sheet, but a preferred method of creating the microstructure is by hot embossing, whereby a hot, patterned cast is pressed into the plastic to create the microstructure. When created in this manner, the tops of the channels will typically be flush with, or slightly raised from, the surface of the sheet.

The channels or pits are preferably approximately the width of the target cells, or slightly wider or narrower in a manner judged by the skilled person as sufficient to provide a suitable attachment point for the target cell. In this instance, the target cell is the cell in cell culture that it is desired should be deposited in the pattern determined by the microstructure. In general, it has been determined that the depth of the channels is about half of the width, thereby allowing sufficient exposure of deposited cells to the culture medium to permit continued growth and differentiation, where this is desired.

The microstructure may be formed on the sheet by a process of hot embossing.

The matrices for the moulding of hot embossing stamps, preferably of PMDS, may be produced by microlithography from a silicon plate, as known to those skilled in the art. In one example, thorough isotropic etching with a resist mask results in all concave recesses being semicircular in cross-section, and having a maximum depth of half of the structural width. Following chemical purification with peroxodisulphuric acid and modification of the silicon surface with monomethyl trichlorosilane as the separating agent, the silicon plate is placed in a mould, coated with monomethyltrichlorosilane as a release agent, and coated with PDMS not yet copolyermerised. After polymerisation at temperatures between 80 and 100°C for an hour, the blank of a hot embossed stamp can be released. The silicon matrix is reusable. After washing the stamp in a 25-fold excess of absolute ethyl alcohol with three changes over three days and treatment in a low-pressure oxygen plasma, the adhesiveness on thermoplastic polymers of the PDMS surface can subsequently be eliminated by chemical modification with heptadecafluoro-1,1,2,2-tetrahydrodecyl)-trimethoxysilane (HTTS). The stamps, or dies, thus produced can be used repeatedly for hot embossing.

A plurality of microstructures may be repeated to form patterns.

A dominant dimension of the microstructure is a similar order of magnitude as a cell.

The dominant dimension of the microstructure is between 1 and 100 µm. The dominant dimension may be: between 20 µm and 40 µm; 35 µm; between 15 µm and 20 µm; or between 5 µm and 15 µm.

It will be appreciated that it may be advantageous to provide side groups and optionally linked substituent molecules on the sheet, either on the sheet as a whole or in the microstructure. These substituents may serve any suitable purpose as desired by the skilled person, including securing cells, marking cells, preventing undesired cells from attaching, providing nutrition, acting as hormones, stimulants or differentiation signals, etc. Some such substituents include, but are not limited to; silanes, isothiocyanates, amino groups, maleimides, and click chemistry additions, such as those sold by Invitrogen. Non-covalent additives for binding purposes or cell differentiation include retinoic acid, biotin, and streptavidin.

The support may be formed from any suitable material, but is preferably one to which the material of the sheet can be fused. It is preferred that the material of the support is a thermoplastic when the material of the sheet is thermoplastic, but there is no requirement for the material of the support to be biodegradable.

It is preferred that the material of the support is non-resorbable, in that it is preferred that it does not degrade to any substantial degree in the presence of the selected cell culture. Thermoplastics used in the food industry for packaging are a preferred group of materials for the support and macrofeatures.

It is preferred that the microstructure of the sheet be created by hot embossing. In such an embodiment, it is preferred that the material of the support is a thermoplastic that has its glass transition temperature above the temperature of the heated die used to imprint the microstructure in the sheet. In this respect, it is preferred that the support has a thickness in the same order of magnitude as the sheet. In one embodiment, the heated die is pressed onto the module, and covers the sheet and at least a portion of the support, thereby ensuring that the whole of the sheet is patterned. It will also be appreciated that a skeleton, or thicker structure, may surround the support, for removal after patterning the sheet, such that a module having a thickness of the same magnitude as that of the sheet may be obtained.

Examples of thermoplastic polymers useful in the present invention include; acrylonitrile butadiene styrene (ABS), poly(methyl methacrylate (PMMA), celluloid, cellulose acetate, cyclic Olefin Copolymer (COC), ethyleneVinyl Acetate (EVA), ethylene vinyl alcohol (EVOH), fluoroplastics, including PTFE, FEP, PFA, CTFE, ECTFE, ETFE, polyoxymethylene (POM, Acetal), polyacrylates, polyacrylonitrile (PAN), polyamide (PA, Nylon), polyamide-imide (PAI), polyaryletherketone (PAEK), polybutadiene (PBD), polybutylene (PB), polybutylene terephthalate (PBT), polycaprolactone (PCL), polychlorotrifluoroethylene (PCTFE), polyethylene terephthalate (PET), polycyclohexylene dimethylene terephthalate (PCT), polycarbonate (PC), polyhydroxyalkanoates (PHAs), polyketone (PK), polyethylene (PE), polyetheretherketone (PEEK), polyetherketoneketone (PEKK), polyetherimide (PEI), chlorinated Polyethylene (CPE), polyimide (PI), polymethylpentene (PMP), polyphenylene oxide (PPO), polyphenylene sulfide (PPS), polyphthalamide (PPA), polypropylene (PP), polystyrene (PS), polysulfone (PSU), polytrimethylene terephthalate (PTT), polyurethane (PU), polyvinyl acetate (PVA), polyvinyl chloride (PVC), polyvinylidene chloride (PVDC), styrene-acrylonitrile (SAN), and copolymers of any combination thereof.

Preferred thermoplastic polymer to form the support include: acrylonitrile butadiene styrene (ABS), polycarbonate (PC), polyethylene (PE), and polypropylene (PP), and their co-polymers.

The support may form the entirety of the macrostructure, and may comprise a skeleton such as described above. The macrostructure may also comprise additional structures, such as areas within the sheet itself that are not connected to the support except *via* the sheet, or sheets. One example of such a structure is a ring that matches a ring in another module, such that a rod or some such may be passed through the holes created by the rings to keep the modules in register, or to pass other items or observation means therethrough.

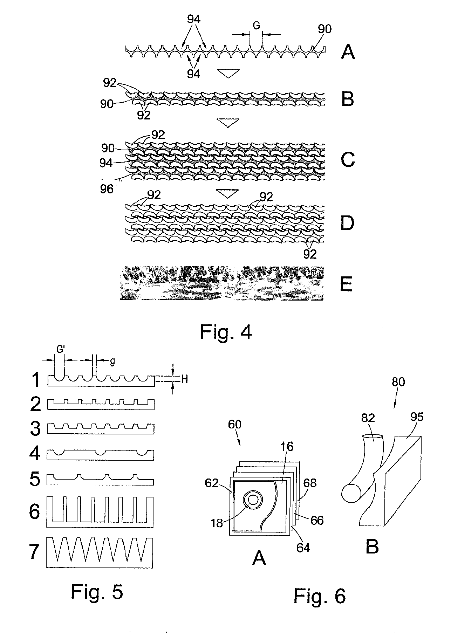

It will be appreciated that the modules may be stacked, and it is preferred that the modules may be stacked in such a fashion that cell cultures associated with the sheets of separate modules may grow to confluence, such as is illustrated in accompanying Fig. 4.

Stacking of the modules of the invention may be of similar modules or of different modules. The modules may vary in size and/or shape. The modules may carry different cell cultures, or cultures of similar cells but with different growth media.

Modules of the present invention may be incubated in the presence of a cell culture by complete immersion in one culture, or may be incubated with a culture on one side and, when the culture has affixed to one side, then the module may be inverted to cultivate an alternative culture on the other side. The module may subsequently be stacked with other modules prepared with desired layers of cells, and a tissue or organ structure may thereby be obtained, or seeded.

In a preferred embodiment, when stacking modules, the macrostructures may suitably be adapted to interact in such a fashion as to be able to form a stable structure, such as by simple clips, or protrusions engaging with recesses. In an alternative, the modules may be secured within suitable apparatus, such as within an irrigated tube, or protrusions may engage recesses, with either being located on or in the apparatus and the opposite being located on the modules, such as a ridge being located in a tube to engage a recess on each module.

As noted above, the macrostructures may comprise a frame, a lug, etc. When the macrostructure comprises a frame, the frame may incorporate lugs to assist with positioning.

In one embodiment, the sheet may comprise two or more sub-sheets comprised of the same or different polymers, each sheet having a microstructure formed on at least one side.

A further aspect of the invention provides a model for cell cultivation comprising a plurality of the modules of the invention arranged, preferably as described above, such that the supports internally define a volume corresponding to all or part of a tissue or organ structure. It will be appreciated that the volume contains multiple sheets, and it is preferred that cell cultures associated therewith are further grown together, preferably to confluence, such as to provide said tissue structure or organ part.

Such structure may provide the basis for actual tissue or organ, by being further grown after removal of the support, or may form the tissue or organ. For example, replacement cartilage may be grown in this manner for knee or vertebrae operations.

The microstructures and the macrostructures may be arranged so that cells cultured on the microstructures form a tissue which corresponds to the body formed by the macrostructures of the stack of the modular elements.

A further aspect of the invention extends to a method of producing a modular element for cell cultivation, the modular element comprising a sheet having a microstructure formed thereon, one or more macrostructures, the macrostructure comprising a macrofeature wherein a dominant dimension of the macrofeature is of a similar size order as the sheet, the method comprising:
providing the sheet;
forming the macrostructure in contact with the sheet by a process of fused deposition modelling.

The microstructure may be formed on the sheet by stamping. A PDMS stamp may be used to stamp the microstructure.

The sheet may be formed by a process of fused deposition modelling.

As noted, the sheet comprises a first surface and a second surface, and a microstructure may be formed on the first surface and a further microstructure formed on the second surface. The microstructure formed on the first surface may be complementary to the microstructure formed on the second surface, or may be different.

In an embodiment, a sheet is formed with a pattern of repeating microstructures, the repeating microstructures each comprising a trough, or channel, having a depth of between 5 µm and 50 µm. Preferably, the depth is between 10 and 40 µm. More preferably, the depth is between 15 and 30 µm. The patterns may have any cross section, and may be convex, concave, V-shaped or U-shaped and may have orthogonal, or sloped walls, or a combination of these shapes and wall characteristics, for example.

A sheet may have a single pattern.

Each surface may have more than one pattern.

In one embodiment, the pattern comprises more than one microstructure.

As noted above, it is preferred that the sheet be made of a material, preferably a polymer, that is capable of attachment to a biological group.

In a further embodiment, one or more module elements comprise two sheets comprised of different polymers, each sheet having a microstructure formed thereon. Preferably, the microstructure when formed as a pattern extends over both sheets. The two sheets may form a common plane.

In a further embodiment, there is provided a body comprising a plurality of modular elements arranged in a stack, a plurality of the modular elements having corresponding macrostructures arranged so that the macrostructures form a body.

The body may be shaped and sized to resemble a structure to which cells would adhere in an animal or human. In this case, the microstructures and the macrostructures may be arranged so that cells cultured on the microstructures correspond to the body formed by the macrostructures of the stack.

The body may be one or more of a vessel, a nerve, bone, cartilage, skin, muscle, or other organ or tissue, or part thereof.

The possible applications of the invention include tissue culture in tissue engineering, as well as the provision of living test structures as a substitute for organs, tissues, and experimental animals in the search for pharmacologically effective substances and in the testing of drugs as part of the testing and approval process. Those skilled in the art are aware that, in signalling within tissues and between organs, the relationship between transmitters and receivers is critical. This relationship is essentially subject to two parameters, the relative number and the geometric arrangement of transmitting and receiving cells. This principle extends to mechanical, electrical, and chemical signals. If a test arrangement is intended to be representative for the system to be characterised, e.g., a tissue plate of heart muscle cells is to represent the heart muscle, then the cell types, their relative number, and their arrangement in native tissue should correspond to the cell types, their relative number, and their arrangement in cloned tissue plates for the best results. This can be achieved with the present invention. Thus, geometric patterns can be filled with heart muscle cells organised similar to tissue, the shape and related electrical signal transduction of which reproduces the typical conditions obtaining in heart diseases. This allows for significantly better characterisation of effects of drugs on the cardiac system than in single cell *in vitro* studies or studies in experimental animals.

Advantages of the present invention include:
1. The material composition modules and their surface structure can be created to the user's specifications, and can be readily produced in quantity;
2. The shape and structure of the modules allow them to be handled in a conventional laboratory environment and to be used in conventional cell culture techniques;
3. The modules have two parallel surfaces that can be equipped with identical or different structural motifs;
4. The modules can be moved, turned, and stacked, and are amenable to mechanical, electrical, optical, and chemical influence by the user; and
5. The modules can be sufficiently translucent to allow for optical microscopy.

Polylactic acid is a preferred biodegradable material of the present invention and is generally used herein to exemplify any biodegradable substance of the invention. Unless otherwise apparent from the context, any mention of Polylactic acid or PLA is to be understood to include reference to any other biodegradable material of the present invention.

More generally, PLA polymer may be obtained as a powder, in preference to pellets, and is preferably surface-activated to increase the concentration of reactive hydroxyl (OH) groups. Reactive OH may be introduced in two locations; either at the ester bonds within the polymer chain (...-O-C(=O)-C-....); or on the aliphatic chains between the ester groups (...-CH₂-CH₂-...).

Basic compounds such as sodium hydroxide or ammonium hydroxide dissolved in aqueous solution, in dimethylformamide (DMF), or in dimethylsulfoxide (DMSO) are capable of opening ester bonds. This creates shorter polymer chains, but with both reactive hydroxylic end groups and reactive acidic end groups (-COOH). Both reactive groups can be used for chemical coupling. This approach keeps the aliphatic parts of PLA unchanged but reduces the entire length of the polymer chain. The amount of ester bond disruption will determine the effect on other physical properties, such as degradation time, solubility, melting point, crystallinity and mechanical stability.

Low pressure ion plasma, with ionised water or ammonium molecules, creates hydroxylic side groups or amino side groups, accordingly, *via* a radical oxidation process at the aliphatic hydrocarbon locations inside the PLA backbone. The reaction products are secondary alcohols or primary or secondary amines (...-CH₂-... => ...-CHOH-... or ...-CH(NH₂)-...). Acidic end groups are also likely to be converted into carboxylic acid amides, and hydroxylic end groups may be converted into primary amines, depending on the ion plasma used.

Other surface activation techniques may also be used, as will be familiar to those skilled in the art.

After basic activation, the polymer will generally be present as a colloid in solution, such as in DMF or DMSO, where these were used, and should be purified to remove traces of reactants, such as to remove traces of sodium hydroxide or ammonium hydroxide, for example. The swollen polymer colloid may be mixed with water or ethanol, such as absolute ethanol, methanol, water, or a comparable polar solvent. The polymer will usually precipitate, and can be washed with an excess of solvent, typically until the pH of the solvent is at or below pH7. Modified polymer powder obtained *via* plasma treatment can normally be used without further washing or purification.

After washing, the purified PLA either production process will normally be in the form of a dry powder, which can be re-swollen or dissolved in a solvent, such as DMF. The powder is preferably not desiccated using high temperatures or under strong vacuum conditions, as this may give rise to re-esterification between the hydroxylic and carboxylic acid groups. A small amount of water may be helpful in any the subsequent silanisation process.

The reactive chemical groups of the modified polymer are suitable subjects for condensation reactions with optionally derivatised silanol groups (-Si(OH)ₙ with n<4). The optional side chains, or substituents, may include: aliphatic and aromatic amines, such as n-aminopropyltriethoxy- (APTES), n-(n-butyl)-3-aminopropyltriethoxy-, n-(6-aminohexyl)aminopropyltriethoxy-, n-methyl-3-aminopropyltriethoxy-, and n-aminoethyl-3-aminopropyltrimethoxy-; amino-group reactive compounds, such as isothiocyanoalkyl (SCN-CHx-...), N-hydroxysuccinimidic or succinomaleic esters; hexyltrichloro-; and methacrylic groups. The above may generally be in the form of ethoxy, chloro, methoxy, or hydroxy silanes, although other forms, or combinations of the foregoing, are also envisaged. The above compounds are hydrolysable in the presence of small amounts of water and the reaction products are typically mono-, di-, and preferably trihydroxysilanes, with the fourth valence carrying the reactive group of interest for later reactions.

Coupling of the resulting, derivatised silane with hydroxy groups on the polymer chain covalently introduces the silanyl-reactive groups into the polymer.

The reaction mix, where the solvent used is DMF, may be heated above 100°C, as DMF boils above 150°C, to remove any water and to promote the condensation reaction. It will be appreciated that any heating will need to take account of any thermolability of the constituent components, such as any isothiocyano-, succinimidic-, maleimidic, and methacrylic- reactive groups. The reaction may also be divided into two subreactions, the first taking place at normal atmospheric pressure and the second at reduced pressure, for example, this having the benefit of removing both water and DMF.

The reaction product is a derivatised polymeric product based on PLA, but comprising reactive side chains and/or end groups. The derivatised PLA is capable of covalently binding amino acids, such as gamma-amino butyric acid, GABA, peptides, such as RGD signalling peptide or oxytocin, and proteins, by reacting with their amino groups, typically the terminal amino group. Other amino-modified polymers, such as amino modified polyethyleneglycols, may be coupled to the polymer to provide graft copolymers.

PLA derivatised with methacrylic-silane groups allows a second reaction step of adding modified acrylic and methacrylic reactive compounds. The reaction can be catalysed using heat sensitive or UV sensitive radical initiators. This way, non-protein compounds lacking amino groups, such as modified carbohydrates, for example inositol-trisphosphate (IP3), or fatty acid derivatives, for example diacylglycerol (DAG) or the terpenoids, for example retinoic acid (RA), can be coupled directly by a non-specific radical mechanism or indirectly *via* preformed methacrylic derivatives.

With fatty acid derivatives and terpenes, it is possible to mix the small compounds with the molten, or dissolved, polymer. For example, PLA can be either dissolved in DMF or kept above its melting point in DMF. When in solution, PLA is in the form of a colloid. In the latter case, a clear solution is obtained. The resulting PLA, obtained after removal of any solvent, is then able to release the small compound, *in situ.*

The resulting derivatised PLA polymer can be extruded to make up polymer filaments useful for fused deposition modelling, or may be thermoformed between two micropatterned stamps, preferably PDMS stamps, to give a micropatterned PLA sheet having two reactive and/or signalling surfaces. In one embodiment, a PLA sheet with a reactive surface may be immersed in a solution of one or more of the above mentioned compounds to introduce, for example, a signalling functionality in a second step.

Any residual active groups may be inactivated by the addition of, for example, an aqueous solution of biotin, thiol-carrying compounds, bovine serum albumin, foetal calf serum, ovalbumin, skimmed milk powder, or small polyamines, such as spermines and spermidines.

The derivatised and micropatterned sheets may then be inoculated with eukaryotic cells. We have performed experiments with the murine embryonic stem cell line P19 and subclones thereof, such as P19Cl6, but any suitable eukaryotic, and preferably mammalian cells may be used. Human stem cells, preferably monpotent, oligopotent, or pluripotent, may be used. Progenitor cells and juvenilised cells, preferably induced pluripotent stem cells iPCs, may be used. Other cell lines, such as those obtained after DNA demethylation obtained, for example, using 5-aza-cytidine, or micro-RNAs, such as miRNA-145, may also be used.

Any suitable cell culture medium may be used. For example, with murine P19, αMEM medium supplemented with FCS, foetal calf serum, pyruvate, L-glutamine and 5-Aza-cytidine, together with cultivation over 5-10 division cycles, results in a P19 cell population having demethylated DNA. Subsequent reduction of the serum content and removal of the 5-aza-cytidine, and transferring the culture onto a sheet of the invention presenting the cell signalling peptide hormone oxytocin on the scaffold sheet, induces differentiation into cardiac myocytes, for example.

In an alternative, presentation or release of retinoic acid by the sheet can induce differentiation into neuron precursor cells.

Repeat washing of undifferentiated cells by a calcium and magnesium depleted medium and subsequent addition of 5-azacytidine treated cells results in a high density coating with a spatially organised cardiac or neuronally differentiated cell monolayer. Differentiation is gradual, and may take a few weeks to reach a final state. It will be appreciated that the combination of differentiating cells and differentiation compounds to make up other differentiated cells are possible, such as endothelial cells, interstitial tissue cells, for example fibrocytes, and epithelial cells.

After inoculation onto the sheet, the cells are rapidly spatially organised by the effect of the micropattern. Any differentiation signal provided by the surface is preferably active over a period of a few hours to a few days. After reaching confluence, the cell coating may be deaggregated either by chemical ester cleavage or by cell metabolism. This progressively removes the PLA material from the cell sheet. The time needed for this removal depends from the crystallinity of the PLA in use and covers a time scale between below one month and more than three months. The use of PLA with longer lifetimes, such as up to 2 years, is envisaged.

The cultivation of inoculated sheets may be performed in bacteriological culture dishes or on other hydrophobic supports, such as a PDMS coating, a Teflon® dish, etc. This prevents the cell population from seeding other surfaces than the sheet substrate and reduces the possibility of false differentiation signals *via* surface contacts. It is preferred to use non-resorbable polymers as the support to make it possible to handle the cell sheet mechanically after the PLA component has been resorbed, or decomposed. Cell sheets can be stacked to set up three dimensional tissue bulk, conserving the spatial organisation of the cells in the constitutive sheets. By combining cell populations differentiated to myocardial muscle cells with endothelial cells, or fibroblasts, muscle bulks with vascular cavities may be constructed.

The combination of differently decorated PLA polymers as a mosaic in one layer by fused deposition modelling a sheet with a FDM printer, for example, printing two or three PLA polymers, and the subsequent spatially restricted individual differentiation of stem cells on this mosaic, allows the preparation of a tissue slice, for instance, after stacking a tissue block, optionally with different tissue types inside one sheet or block.

### Brief Description of the Drawings

Aspects of the present invention will now be further described, by way of example only, with reference to the accompanying Figures, in which:
Figure 1 is a schematic illustration of a top view of a modular element according to an embodiment of the invention;
Figure 2 is a schematic illustration of a side view of the modular element of Figure 1;
Figure 3 is a view of a plurality of modular elements constructed according to an embodiment of the invention;
Figure 4 is a series of drawings showing the culturing of cells on a modular element according to an embodiment of the invention;
Figure 5 illustrates a plurality of sheets of modular elements of corresponding embodiments of the invention;
Figure 6 illustrates a scaffold of modular elements according to an embodiment of the invention; and
Figure 7 is a flow diagram of a method of constructing a modular element, and a scaffold, according to embodiments of the invention.

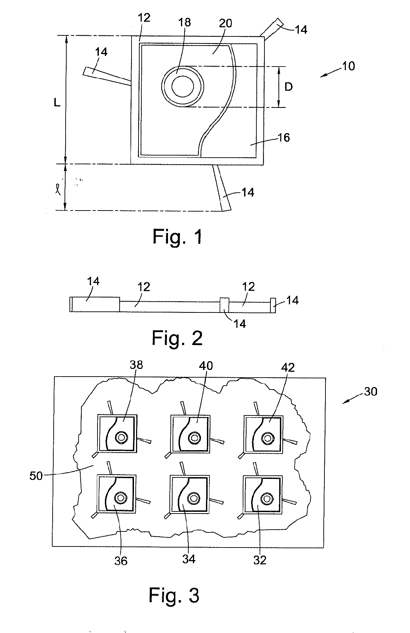

Figure 1 illustrates a modular element 10 according to an embodiment of the invention. The modular element 10 comprises a frame 12 to which three lugs 14 are attached. The frame 12 surrounds a sheet 20 on one side and an undulating formation 16 on the other. Sheet 20 surrounds an annular formation 18.

In this embodiment, the frame 12, lugs 14, undulating formation 16 and the annular formation 18 are macrostructures. The frame 12 is square in this embodiment having a length L, as shown in Figure 1. Lugs 14 have a perpendicular projection from the frame 12 of a distance **ℓ**, and annular formation 18 has a diameter, D. Undulating formation 16 has a length which is substantially similar to that of the frame 12 (i.e. length L).

The length L of frame 12, distance **ℓ** of lug 14, diameter D of annular formation 18 and length L of undulating L are dominant dimensions of those macrofeatures in that they describe a predominant feature of each of the macrofeatures. It will be appreciated that what is to be considered as a dominant dimension of a macrofeature will depend on the size, shape and extent of the particular macrofeature concerned. In an embodiment, the dominant dimension is a linear extent of the macrofeature. In a further embodiment, the dominant dimension is the longest dimension of the macrofeature, or a dominant portion of the macrofeature.

In the embodiment illustrated, L is 1 cm, **ℓ** is 0.4 cm and D is 0.4 cm. However, it will be appreciated that the actual values of these dimensions are not essential to embodiments of the invention. These dimensions are, however, of the same order of magnitude as the sheet 20. In this respect, although the same order of magnitude for the dominant dimension of the macrofeature is specified, it will be appreciated that this does not exclude macrofeatures having a dominant dimension less than ten times the size of a dimension of the sheet, or smaller. In certain embodiments of the invention, the dominant dimension of the macrofeature is at least four times larger than a dominant dimension of a microstructure of the sheet (*c.f. infra*).

Sheet 20 has a pattern of microstructures formed on a surface thereof, in the manner described below.

The sheet 20 is formed from a first thermoplastic polymer; the frame 12, lugs 14, undulating formation 16 and annular formation 18 are formed from a second thermoplastic polymer. The sheet may be made from PLA, and the support, or frame, from ABS, but alternatives for each are well known in the art and as exemplified above.

Figure 2 is a side elevation of Figure 1.

Figure 3 shows a block 30 of six modular elements 36, 38, 40, 42, 44 and 46 formed on a common, pre-formed support, in the form of a patterned sheet 50. The sheet 50 is similar to the sheet 20 with the obvious difference that the sheet 50 forms the sheet for each of the six modular elements 36, 38, 40, 42, 44 and 46. In use, the modular elements 36, 38, 40, 42, 44 and 46 are removed from the common sheet 50, each modular element retaining a portion of the sheet 50.

Figure 4A is a cross section through a sheet 90, in the absence of cell culture or support. Sheet 90 may be formed by sheet 20 from Figure 1, for example.

Sheet 90 of Figure 4A has a pattern formed thereon. The pattern of sheet 90 is formed by a repetition of grooves 94 formed on either side of the sheet 90. In the embodiment illustrated, the grooves 94 may be formed by stamping with a PDMS stamp. However, in general, the patterns on a sheet of the invention may be formed by any one, or a combination of, soft lithography, self assembly, vapour deposition and photolithography.

As illustrated in Figure 4A, the grooves 94 have a width G. In this embodiment, G is suitably 35 µm. In this embodiment the grooves 94 form microstructures of the sheet 90 and G is a dominant dimension of the grooves 94 insofar as they define the manner of the pattern formed by the repeated grooves 94. In this respect, it will be appreciated that the grooves 94 also comprise a length which will be significantly larger than the width, but the width is to be considered the dominant dimension as this defines the pattern.

In the embodiment illustrated, the dominant dimension of the microstructure may be of the order of 35 µm. Embodiments of the invention are not so limited; what is important for embodiments is that the dominant dimension is the same, or a similar, order of magnitude as a cell. It will be appreciated however that cells may vary substantially in size and therefore, the dominant dimension of the microstructure of the sheet may be dependent on the type of cell to be cultivated on the sheet. In embodiments, the dominant dimension may be between 20 µm and 40 µm; 35 µm; between 15 µm and 20 µm; or may be between 5 µm and 15 µm.

Figure 4B illustrates the manner in which cells 92 are cultivated in the grooves 94. Figure 4C illustrates the manner in which a plurality of sheets of cells 92 may be formed by situating a number of sheets 90, 94 and 96 adjacent one another, and illustrates stacking of the modules as discussed *supra.*

Figure 4D illustrates the arrangement of cells 92 when the sheets 90, 94 and 96 have degraded. It will be appreciated that the sheets are preferably comprised of a biodegradable polymer and that once the sheet has degraded, the cells which were cultured on the surface of the sheets remain. In this manner, complex, multi-cellular structures may be constructed from a plurality of modular elements of the type described above.

Figure 4E is a micrograph of a comparative tissue sample.

Figure 5 illustrates a number of microstructures which may be formed on the surface of a sheet. Figure 5.1 illustrates a microstructure in the form of a U-shaped groove with a dominant dimension G', which may be 35 µm, for example, and a second dimension g, which may be 5 µm, for example. A third identified dimension is the depth of the grooves H, which in this embodiment would be 15 µm. It will be appreciated that g or H may, in addition or alternatively, be considered as a dominant dimension of the microstructure here forming the pattern of the sheet.

Figures 5.2, 5.3, 5.4 and 5.5 illustrate alternate microstructures with corresponding microstructure which may be used for cell cultivation. Figures5.6 and 5.7 illustrate microstructure used for purposes other than cell cultivation. For example, the sheet of Figure 5.6 may be used for microfluidic applications. Where the sheet is not used for cell cultivation, the size of the microstructure may be determined by the function of the sheet.

All of the sheets illustrated in Figure 5 have a microstructure formed on only one surface. However, in further embodiments, the same, or a different, microstructure may be produced on the obverse side of the sheet. Furthermore, the microstructure may be repeated to form a pattern, as shown in Figure 5. It will be appreciated that the repetition of the microstructure may be regular, as shown, but in further embodiments the repetition may be variable, for example, it may comprise grooves in various directions, or may be contoured to define more complex patterns or areas, such as spirals, concentric circles, or even angled shapes or latticed patterns.

In a further embodiment, the sheet may be formed from two sub-sheets joined together. So, with reference to Figure 1, the undulating formation 16 may, instead, be formed of a different polymer to that of the sheet 16, or may be formed of the same polymer, and have a pattern formed thereon. The pattern may be formed by a different, or the same microstructure. The two sub-sheets are then adhered to one another to form a single, discontinuous sheet.

Figure 6A illustrates a plurality of modular elements 62, 64, 66 and 68 stacked together to form a structure 60. Each of the modular elements 62, 64, 66 and 68 comprise two macrofeatures: annular formation 18 and undulating formation 16.

According to embodiments of the invention, a plurality of the modular elements having different shaped and sized macrofeatures may be combined in a stack so that the successive macrofeatures form a body. Figure 6B illustrates two bodies: a vessel 82 and a bone fragment 84 formed by the plurality of successive macrofeatures of the modular elements 62, 64, 66 and 68 of Figure 6A. For the sake of clarity, the other macrofeatures such as the frames, and the sheets, of the modular elements are not illustrated in Figure 6B.

It will be appreciated that any biological macrostructure may be modelled in this way: by a plurality of modular elements, each representing a cross-section of the macrostructure. Therefore, the cells which are cultivated on the sheets of the modular elements 62, 64, 66 and 68 are preferably cells corresponding to tissue types associated with the bodies (or biological macrostructures) formed by the plurality of macrofeatures of the modular elements.

Advantageously therefore, embodiments of the invention facilitate the cultivation of tissues and their corresponding macrostructures which allows for the study of how these tissues interact with these macrostructures. Similarly, where embodiments of the invention are concerned with sheets used for purposes other than cell generation, the interaction with the micro- and macrofeatures may be observed in scaffolds according to embodiments of the invention.

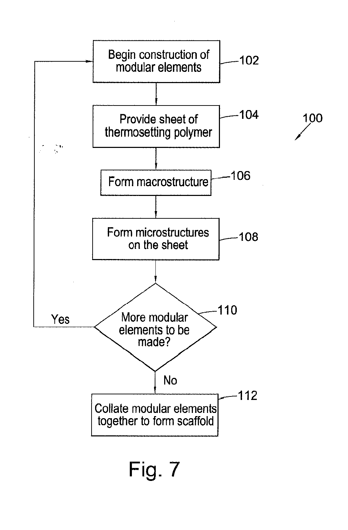

Figure 7 illustrates a flow diagram 100 of a method of constructing one or more modular elements, and a structure, according to embodiments of the invention. The method 100 commences at step 102 where the construction of the modular elements commences. This step will comprise all the preliminary steps needed to construct a specific modular element. For example, this step will involve the loading of the specification (in this embodiment in the form of a CAD file for the modular element).

At the next step, step 104, a sheet is provided. In an embodiment, the sheet is prepared and is pre-formed with the microstructure as described above.

In the embodiment illustrated, the sheet is formed by a process of fused deposition modelling (FDM) at step 104. Any appropriate FDM method may be used, but in an embodiment, thermoplastic polymer FDM is used with any one of the materials discussed above. In embodiments of the invention, the FDM printer type used is "3D Touch, triple head version" sold by 3dsystems.

The macrostructures are then formed on the modular element in step 106. In this embodiment, the macrostructures are also formed by FDM using a second thermoplastic polymer. The melting point of the second thermoplastic polymer is higher than that of the first thermoplastic polymer. Therefore, when the macrostructures are formed on the sheet, a localised melting of the sheet will occur, helping to ensure that there is adhesion between the macro- and micro-structures of the modular element occur during manufacture. This helps to prevent gaps arising between the different structures formed from different thermoplastic polymers.

In further embodiments, the macrostructures are formed from two or more different thermoplastic polymers.

In the following step, step 108, the microstructure is formed on the sheet. It will be appreciated that this step is optional, and will not be carried out in those embodiments where the sheet has been pre-prepared. However, in this embodiment, the microstructure is formed by hot embossing with the use of a PDMS stamp.

Since the thermoplastic polymer of the sheet has a lower melting point than that of the macrofeatures, the stamping is carried out so that the macrofeatures will not be affected by the stamping procedure. Therefore, advantageously, it is possible to select the features which are provided with the patterning, and therefore the features on which, for example, cells are cultivated.

At step 110 a decision is made as to whether more modular elements are to be constructed. As described above, embodiments of the invention extend to bodies formed by a scaffold of successive modular elements where the macrostructures of successive modular elements differ from one another. Therefore, if it is decided at step 110 that there are further modular elements to be made, the process will proceed back to the initiation step 102 and here the specification for the next modular element will be loaded.

If it is decided at step 110 that no further modular elements are required, the process will proceed to step 112 where successive modular elements are placed together in a stack to form a structure. It will be appreciated that any appropriate method of adhering modular elements to one another may be used in this step.

Although the modular elements described and illustrated here are in the form of a straight plane, it will be appreciated that the sheets of the modular elements may be bent or folded to form more complex shapes.

It will be appreciated by the person skilled in the art that various modifications may be made to the above described embodiments without departing from the scope of the present invention.

## Claims

1. A modular element, adapted to serve as a scaffold for cell cultivation, comprising a degradable, preferably biodegradable, thermoplastic sheet and a support structure therefor, said degradable sheet having a microstructure on one or both sides, and wherein said modular element is stackable.

2. A module according to claim 1, wherein the support is in the form of a surround for the sheet, with the sheet corresponding to a cross-section of a tissue or organ whereby, by selecting appropriate cross-sections and cell cultures, a plurality of said modules can be arranged such that said sheets and any cell cultures associated therewith form the shape of a cell mass desired.

3. A module according to claim 1 or 2, wherein the sheet is constructed from a plastic selected from: aliphatic polyesters such as polyhydroxyalkanoates (PHAs), for example poly-3-hydroxybutyrate (PHB), polyhydroxyvalerate (PHV) and polyhydroxyhexanoate (PHH); polylactic acid (PLA); polybutylene succinate (PBS); polycaprolactone (PCL); polyanhydrides; polyvinyl alcohol (PVA); starch derivatives; and thermoplastic cellulose esters, such as cellulose acetate, cellulose acetate butyrate, cellulose propionate, and ethyl cellulose, preferably PLA, poly(lactic-co-glycolic acid) (PLGA), polylactic acid and polycaprolactone (PLA-PCA), PLA-polyurethane (PLA-PU), and any copolymers thereof.

4. A module according to any preceding claim, wherein the microstructure is adapted for use in directing, at least to some extent, growth of any cells in cell culture to which the microstructure is exposed.

5. A module according to any preceding claim, wherein the smaller dimensions of features of the microstructure intended for interaction with cells of said cell culture are similar to the dimensions of said cells, preferably approximately the width of the target cells, or slightly wider or narrower,

6. A module according to any preceding claim, wherein the support is formed from a thermoplastic having a higher melting point than that from which said sheet is formed.

7. A module according to any preceding claim, wherein side groups and/or optionally linked substituent molecules are provided on and/or within said sheet, such as to secure cells, mark cells, prevent undesired cells from attaching, provide nutrition, act as hormones, stimulants or differentiation signals, etc.

8. A module according to claim 7, wherein substituents are selected from; silanes, isothiocyanates, amino groups, maleimides, click chemistry additions, retinoic acid, biotin, and streptavidin.

9. A module according to any preceding claim, wherein a plurality of microstructures are repeated to form patterns.

10. A method of producing a modular element for cell cultivation as defined in any preceding claim, comprising fusing a degradable, preferably biodegradable, thermoplastic sheet to a frame therefor constructed from a thermoplastic material having a higher glass transition temperature than that of said sheet, and forming at least one microstructure on said sheet by hot embossing.
